# EUROPÄISCHE PATENTANMELDUNG

(11) **EP 2 772 142 A1**
(43) Veröffentlichungstag der Anmeldung: **03.09.2014**
(21) Anmeldenummer: 13157041.8
(22) Anmeldetag: 27.02.2013
(51) Int. Cl.: A23L 1/221, C07C 47/58, C12P 7/24

(54) **Vanillin**

(71) Anmelder: Symrise AG, 37603 Holzminden (DE)
(72) Erfinder: Kindel, Günter, 37671 Höxter (DE); Hilmer, Jens-Michael, 37603 Holzminden (DE); Gross, Egon-Eduard, 37603 Holzminden (DE)
(74) Vertreter: Fabry, Bernd

(57) **Zusammenfassung**

Vorgeschlagen wird synthetisches Vanillin in einer neuen Geschmacksqualität, welches man erhalten kann, indem man
(a) einen Rückstand aus der Maisverarbeitung mit Alkalien oder esterspaltenden Enzymen behandelt und dabei das darin enthaltende γ-Oryzanol zu Ferulasäure und Sterolen hydrolysiert,
(b) die Ferulasäure aus der Mischung isoliert,
(c) die isolierte Ferulasäure zusammen mit Mikroorganismen vom Typ *Amycolatopsis sp.* DSM 9991 und/oder sp. 9992 in einem Kulturmedium fermentiert, und
(d) nach Beendigung der Reaktion das gebildete Vanillin isoliert.

## Beschreibung

### Gebiet der Erfindung

Die Erfindung befindet sich auf dem Gebiet der Aromastoffe und betrifft Vanillin in einer neuen Geschmacksqualität, ein Verfahren zur seiner Herstellung, Stoffgemische, die es enthalten sowie deren Verwendung.

### Stand der Technik

Vanillin ist ein wichtiger, in der Nahrungs- und Genussmittel-Industrie viel verwendeter Aromastoff. Bislang wird es hauptsächlich aus dem Lignin der Sulfitlaugen, gelegentlich auch durch Oxidation von Eugenol oder Isoeugenol auf chemischem Wege hergestellt. Das auf diese Weise erhaltene Vanillin hat jedoch den Nachteil, dass es im lebensmittelrechtlichen Sinne kein Naturstoff ist und deshalb auch nicht als natürlicher Aromastoff bezeichnet werden darf.

Der natürliche Aromastoff Vanillin ist bisher nur durch Extraktion von Vanilleschoten erhältlich; das auf diese Weise erhaltene natürliche Vanillin ist jedoch sehr teuer. Natürliche Aromastoffe sind chemisch definierte Stoffe mit Aromaeigenschaften, gewonnen durch geeignete physikalische Verfahren (einschließlich Destillation und Extraktion mit Lösungsmitteln), durch enzymatische oder mikrobiologische Verfahren aus Ausgangsstoffen pflanzlicher oder tierischer Herkunft, die als solche verwendet oder mittels herkömmlicher Lebensmittelzubereitungsverfahren (einschließlich Trocknen, Rösten, Fermentieren) für den menschlichen Verzehr aufbereitet werden.

Verschiedene andere Verfahren zur Herstellung von natürlichem Vanillin mit unterschiedlichen Mikroorganismen und Enzymen sind in der Zwischenzeit veröffentlicht worden, liefern aber bislang aufgrund der nur recht geringen Ausbeuten bzw. Konzentrationen keine Produkte auf dem Markt. Beispiele für einschlägige Verfahrens des Stands der Technik finden sich etwa in den Druckschriften EP 0405197 A1 (Haarmann & Reimer), EP 0453368 A1 (Pernod Ricard), EP 0542348 A1 (Quest), oder US 5,128,253 (Gen Foods).

Aus der EP 0761817 B1 (Haarmann & Reimer) ist ebenfalls ein Verfahren zur Herstellung von Vanillin bekannt, bei dem man Ferulasäure aus Eugenol in Gegenwart von bestimmten Bakterien zu Vanillin fermentieren kann. Die Ausbeuten sind dabei deutlich höher als aus anderen Verfahren bekannt, jedoch ist die sensorische Beurteilung der Fermentationsprodukte unbefriedigend.

Die Aufgabe der vorliegenden Erfindung hat somit darin bestanden, Vanillin auf biotechnologischem Wege zur Verfügung zu stellen, das in seiner sensorischen Beurteilung dem natürlichen, aus der Vanilleschote gewonnen Vanillearoma weitgehend entspricht und damit eine kostengünstige und vom Konsumenten akzeptierte Alternative darstellt.

### Beschreibung der Erfindung

Gegenstand der Erfindung ist synthetisches Vanillin in einer neuen Geschmacksqualität, welches dadurch erhältlich ist, dass man
(a) einen Rückstand aus der Maisverarbeitung mit Alkalien oder esterspaltenden Enzymen behandelt und dabei das darin enthaltende γ-Oryzanol zu Ferulasäure und Sterolen hydrolysiert,
(b) die Ferulasäure aus der Mischung isoliert,
(c) die isolierte Ferulasäure zusammen mit Mikroorganismen vom Typ *Amycolatopsis sp.* DSM 9991 und/oder sp. 9992 in einem Kulturmedium fermentiert, und
(d) nach Beendigung der Reaktion das gebildete Vanillin isoliert.

Ein weiterer Gegenstand der Erfindung betrifft ein analoges Verfahren zur Herstellung von Vanillin, bei dem man
(a) einen Rückstand aus der Maisverarbeitung mit Alkalien oder esterspaltenden Enzymen behandelt und dabei das darin enthaltende γ-Oryzanol zu Ferulasäure und Sterolen hydrolysiert,
(b) die Ferulasäure aus der Mischung isoliert,
(c) die isolierte Ferulasäure zusammen mit Mikroorganismen vom Typ *Amycolatopsis sp.* DSM 9991 und/oder sp. 9992 in einem Kulturmedium fermentiert, und
(d) nach Beendigung der Reaktion das gebildete Vanillin isoliert.

Obschon grundsätzlich bekannt ist, dass synthetisch gewonnenes Vanillin je nach Ausgangsstoff und Verfahren infolge der Nebenbestandteile eine etwas andere Geschmacksnote besitzt, wurde überraschend gefunden, dass Ferulasäure, die aus Mais gewonnen wird in Kombination mit einem Fermentationsverfahren, das mit speziellen Bakterien vom Typ der Actinomyceten im Ergebnis zu einer neuen Vanillinqualität führt, die die eingangs geschilderten Anforderungen in hervorragender Weise erfüllt.

Insbesondere die Auswahl von Mais und vorzugsweise von Maiskleie als Quelle für die als Zwischenprodukt gewonnene Ferulasäure war in keiner Weise naheliegend, da es sich um einen Rohstoff handelt, der aufgrund seines vergleichsweise geringen Gehaltes an der Ferulasäurevorstufe, dem γ-Orizanol bislang kaum in Betracht gezogen worden ist. Auch hat sich herausgestellt, dass die Auswahl der Mikroorganismen für die sensorische Qualität des Fermentationsproduktes ausschlaggebend ist: die hervorragende Geschmacksqualität wird ausschließlich mit den beiden oben genannten Amycolatopsis Stämmen erzielt. Die Kombination aus Rohstoffquelle und speziellem Verfahren ist im Stand der Technik nicht enthalten und wird durch diesen zur Lösung der oben beschriebenen Aufgabe auch nicht nahe gelegt.

### Hydrolyse

Wie eingangs erläutert, ist die Gewinnung von Ferulasäure aus Rückständen der Reis- oder auch Maisproduktion grundsätzlich bekannt. In der Monographie von Berger "Flavours & Fragrances" **(bitte nähere Angaben: Verlag, Erscheinungsjahr)** wird im Kapitel 23.4 "Microbial Flavour Production" im Schaubild auf Seite 532 erläutert, dass durch Behandlung dieser Ausgangsstoffe mit geeigneten Esterasen Ferulasäure gewonnen werden kann, die dann mit nicht näher definierten Amycolatopsis-Stämmen in Vanillin umgewandelt werden kann.

Stärkehaltige Pflanzen, wie Kartoffeln, Mais und vor allem Reis, enthalten in unterschiedlichen Mengen γ-Oryzanol, 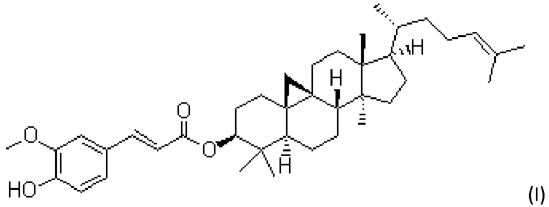 einen Ester der Ferulasäure mit unterschiedlichen Phytosterolen, wie er in der Strukturformel (I) oben exemplarisch dargestellt ist. Durch Behandlung mit Alkalien oder esterspaltenden Enzymen wird der Ester hydrolysiert bzw. verseift und die Ferulasäure freigesetzt. Vorzugsweise erfolgt dieser Schritt als enzymatische Hydrolyse. Als Enzyme kommen für diesen Zweck bevorzugt Ferulasäureesterasen in Frage.

Die Hydrolyse bzw. Verseifung kann dabei in an sich bekannter Weise erfolgen, wie dies beispielsweise für die Herstellung von Ferulasäure aus Reiskleie aus der EP 0503650 B1 (Wakayama) bekannt ist. Dabei kommen etwa Natrium- oder Kaliumhydroxid oder auch entsprechende andere basische Alkalisalze zum Einsatz. Da die Rückstände im Wesentlichen wasserunlöslich sind empfiehlt es sich, ein geeignetes Lösungsmittel, beispielsweise Ethanol mitzuverwenden. Die alkalische Hydrolyse wird in der Regel drucklos bei Temperaturen im Bereich von 90 bis 100 °C und pH-Werten von wenigstens 10 durchgeführt, während die enzymatische Variante deutlich niedrigere Temperaturen und ein eher neutrales Milieu erlaubt, die dem Aktivitätsoptimum der Esterasen entsprechen und typisch bei 28 bis 35 °C und pH 7 bis 8 liegen. Die Reaktionszeiten liegen in der Regel bei etwa 5 bis 24, vorzugsweise etwa 8 bis 20 und insbesondere etwa 10 bis 12 h.

Nach der Hydrolyse liegt die Ferulasäure als Salz vor. Durch Zugabe beispielsweise von verdünnter Schwefelsäure und Einstellen eines pH-Wertes von etwa 2 wird die Säure freigesetzt und fällt bei Temperaturen unterhalb von 30 °C aus. Das Präzipitat kann abfiltriert, gewaschen und gegebenenfalls aus heißem Wasser umkristallisiert werden, wobei die Säure in praktisch reinem Zustand erhalten wird und ohne weitere Aufreinigung in die Fermentation eingesetzt werden kann.

### Fermentation

Bei der Fermentation kommt es zur Umwandlung von Ferulasäure (II) zu Vanillin (III):

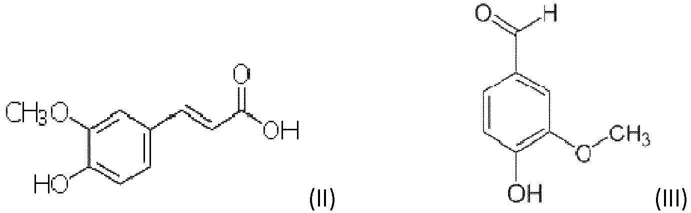

Die Fermentation wird in Gegenwart von Mikroorganismen des Typs *Amycolatopsis species* aus der Gattung Pseudonocardia durchgeführt; entsprechende Stämme sind bei der Deutschen Sammlung für Mikroorganismen und Zellkulturen GmbH in Braunschweig unter den Nummern DSM 9991 und DSM 9992 hinterlegt. Aufgrund der chemotaxonomischen Untersuchungsergebnisse wurden beide Isolate der Gattung Amycolatopsis (Familie Pseudonocardiaceä) zugeordnet. Die Identifizierung basierte auf folgenden fünf Merkmalen: 1. Diagn. Aminosäure des Peptidoglycans: meso-Diaminopimelinsäure; 2. Diagn. Zucker aus Ganzzellhydrolysaten: Arabinose und Galaktose; 3. Mycolsäuren: fehlen; 4. Menachinone: MK - 9 (H4); 5. Fettsäuremuster: Iso/Anteiso- + 10methyl-verzweigte gesättigte und ungesättigte Fettsäuren plus 2-Hydroxy-Fettsäuren. Dieses Fettsäuremuster ist diagnostisch für Vertreter der Gattung Amycolatopsis. Da DSM 9991 und DSM 9992 auch das typische Erscheinungsbild der Vertreter der Gattung Amycolatopsis zeigen - beige bis gelbes Substratmycel und auf einigen Medien auch ein feines weißes Luftmycel - wird die Zuordnung von DSM 9991 und DSM 9992 zur Gattung Amycolatopsis zusätzlich durch die Morphologie unterstützt.

Die Mikroorganismen können in einem üblichen Kulturmedium in für die Kultivierung von Mikroorganismen üblicher Weise kultiviert werden. Das Substrat kann zu Beginn der Inkubation, während oder nach Abschluss des Wachstums auf einmal oder über einen längeren Zeitraum verteilt zugegeben werden. Die Menge an Ferulasäure wird dabei vorteilhaft so bemessen, dass die Konzentration der Verbindung in der Kulturbrühe 80 g/l, vorzugsweise 15 g/l nicht überschreitet. Der Verlauf der Umsetzung kann durch Bestimmung des Ausgangsmaterials und des Produkts in der Kulturbrühe mittels Hochdruckflüssigkeitschromatographie verfolgt werden. Nachdem die optimale Menge an Vanillin entstanden ist, wird dieses durch bekannte physikalische Verfahren wie Extraktion, Destillation oder Chromatographie aus der Kulturbrühe isoliert. Das so erhaltene Rohprodukt kann durch weitere Schritte gereinigt werden.

Die Kultivierung der Mikroorganismen kann in synthetischen, halbsynthetischen oder komplexen Kulturmedien erfolgen. Diese Kulturmedien enthalten vorzugweise Kohlenstoffquellen, Stickstoffquellen, anorganische Salze und gegebenenfalls Spurenelemente und Vitamine. Als Kohlenstoffquellen können z.B. Zucker wie Glucose, Zuckeralkohole wie Glycerin oder Mannit, organische Säuren wie Zitronensäure oder komplexe Gemische wie Malzextrakt, Hefeextrakt, Casein oder Caseinhydrolysat dienen. Beispiele für geeignete Stickstoffquellen sind anorganische Stickstoffquellen wie Nitrate und Ammoniumsalze und organische Stickstoffquellen wie Hefeextrakt, Sojamehl, Baumwollsaatmehl, Casein, Caseinhydrolysat, Weizengluten und Maisquellwasser. Als anorganische Salze können beispielsweise unter anderem Sulfate, Nitrate, Chloride, Carbonate und Phosphate von Natrium, Kalium, Magnesium, Calcium, Zink und Eisen verwendet werden.

Die Kultivierungstemperatur liegt vorzugsweise im Bereich von 10 bis 55 °C, besonders bevorzugt im Bereich von 35 bis 45 °C. Der pH-Wert des Mediums beträgt bevorzugt 3 bis 9, insbesondere 4 bis 8. Die Kultivierung kann entweder in geeigneten Schüttelapparaturen oder in Fermentern mit Mischeinrichtung erfolgen. Bei der Kultivierung ist für eine ausreichende Belüftung Sorge zu tragen. Die Kultivierung kann batchweise, halbkontinuierlich oder kontinuierlich durchgeführt werden. Die Kulturdauer bis zum Erreichen einer maximalen Produktmenge liegt zwischen 4 und 120 Stunden nach dem Animpfen der Kultur. Um die Mikroorganismen vor der toxischen Wirkung der eingesetzten bzw. gebildeten Substanzen zu schützen, kann es vorteilhaft sein, den Kulturmedien Adsorptionsmittel zuzusetzen, z.B. Aktivkohle oder Adsorberharze wie TM Amberlite XAD-2, TM Amberlite XAD-7, XAD-16, TM Lewatit OC 1062 oder OC 1064.

### Gewerbliche Anwendbarkeit

Ein weiterer Gegenstand der Erfindung betrifft eine Stoffmischung, enthaltend
(a) Vanillin und
(b) Vanillylvanillat,
welche die Komponenten (a) und (b) beispielsweise im Gewichtsverhältnis 1:99 bis 99:1, vorzugsweise etwa 5:95 bis 95:5, besonders bevorzugt etwa 25:75 bis 75:25 und vornehmlich etwa 40:60 bis 60:40 enthält. Vorzugsweise wurde das Vanillin dabei nach der eingangs beschriebenen Verfahrensweise hergestellt. Die Kombination der beiden Komponenten (a) und (b) führt zu einer synergistischen Verstärkung des Vanillegeschmackes.

### Nahrungsmittel

Weiterhin umfasst die Erfindung Nahrungsmittel, enthaltend Vanillin erhältlich gemäß der oben geschilderten Herstellungsweise oder die Stoffmischung enthaltend die Komponenten (a) und (b) wie oben erläutert.

### Aromastoffe

Die erfindungsgemäßen Nahrungsmittel können als fakultative Komponente (c) einen oder mehrere Aromastoffe enthalten.

Typische Beispiele umfassen: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Besonders bevorzugt sind die folgenden Aromastoffe, da sie einen milchig-sahnigen und Mundfülle gebenden und/oder einen süß-karamelligen Geschmackseindruck vermitteln: Diacetyl, Acetoin, Benzaldehyd, Furaneol, Heliotropin, Ethylvanillin, Ethylvanillinisobutyrat (= 3-Ethoxy-4-isobutyryloxybenzaldehyd), Furaneol® (2,5-Dimethyl-4-hydroxy-3(2H)-furanon) und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalalcton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd.

### Konfektioniert Produkte

Vorzugsweise handelt es sich bei den Nahrungsmittel um Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Ein weiterer Gegenstand der Erfindung betrifft ein Verfahren zur Aromatisierung von Nahrungsmitteln, bei dem man diesen eine geschmacklich wahrnehmbare Menge Vanillin erhältlich nach der oben geschilderten Herstellungsweise oder eine Stoffmischung enthaltend die Komponenten (a) und (b) wie oben erläutert zusetzt. Eine wahrnehmbare Menge entspricht beispielsweise einer Konzentration von etwa 0,001 bis etwa 0,1 g/kg, vorzugsweise etwa 0,01 bis 0,05 g/kg

Ebenfalls beansprucht wird die Verwendung von Vanillin erhältlich nach der oben geschilderten Herstellungsweise oder der Stoffmischung enthaltend die Komponenten (a) und (b) wie oben beschrieben als Aromakomponenten für Nahrungsmittel.

### Beispiele

### Herstellbeispiel H1

### Herstellung von Vanillin aus Maiskleie

### A. Hydrolyse

100 g dunkelgefärbter Maiskleie wurden in einen 500 ml Dreihalskolben gegeben und mit 5 g einer auf einem Träger immobilisierten Ferulasäureesterase, 20 ml Wasser und 20 ml Isopropylalkohol versetzt. Die Mischung wurde etwa 8 h bei 32 °C gerührt und dann mit 100 ml Hexan versetzt, um alle unpolaren Anteile in Lösung zu bringen. Die organische Phase wurde abgetrennt, die wässrige Lösung mit verdünnter Schwefelsäure versetzt und ein pH-Wert von etwa 2 eingestellt, bei dem die Ferulasäure ausfiel. Es konnten 5 g Ferulasäure mit einer Reinheit von etwa 80 % abfiltriert werden. Diese Menge wurde in heißem Wasser gelöst und durch erneutes Abkühlen umkristallisiert. Es wurden 3,5 g Ferulasäure mit einer Reinheit von 98 % erhalten.

### B. Fermentation

Ein 500 ml Erlenmeyerkolben mit seitlichem Einstich wurde mit 100 Medium, bestehend aus 1 g Malzextrakt, 0,4 g Glucose und 0,4 g Hefeextrakt ad 100 mit Wasser aufgefüllt und anschließend 20 min bei 121 °C sterilisiert. Nach dem Abkühlen wurde der Kolben mit 200 µl einer eingefrorenen Glycerinkultur *Amycolatopsis sp.* DSM 9991 beimpft. Die Kultur wurde auf einer rotierenden Schüttelmaschine bei 45 °C und 100 Upm inkubiert. Nach 24 h wurde die Kultur zur Beimpfung des Produktionsmediums verwendet.

Acht 500 ml Erlenmeyerkolben mit seitlichem Einstich wurden mit je 100 ml Medium (4 g/l Glucose, 10 g/l Malzextrakt) gefüllt und anschließend 20 min bei 121 °C sterilisiert. Nach dem Abkühlen wurden die Kolben mit je 2 ml einer Kultur von *Amycolatopsis sp.* DSM 9991 (wie oben beschrieben) angeimpft. Die Kulturen wurden auf einer rotierenden Schüttelmaschine bei 37 °C und 100 Upm inkubiert. 16 h nach dem Animpfen wurden 20 ml, nach 24 h 409 ml und nach 44 h nochmals 10 ml einer sterilisierten Ferulasäurelösung aus Teil A des Beispiels zu jedem Kolben zugegeben. Nach 46 h wurde die Kulturbrühe der Kolben vereinigt und der Gehalt an Vanillin und nicht umgesetzter Ferulasäure bestimmt: Vanillin: 93,6 %; nicht umgesetzte Ferulasäure: 1.250 ppm.

### Anwendungsbeispiel 1, Vergleichsbeispiele V1 bis V4

### Sensorische Beurteilung

Natürliches Vanillin sowie Vanillin aus verschiedenen Rohstoffquellen und Herstellverfahren wurde auf seine Zusammensetzung untersucht und geschmacklich beurteilt. Die sensorische Beurteilung erfolgte durch ein Panel von 5 geschulten Personen. Zu Grunde gelegt wurde eine Matrix bestehend aus einer 5 Gew.-%igen Zuckerlösung und eine Einsatzkonzentration von 30 ppm. Neben der subjektiven Geschmacksbeschreibung wurde die Ähnlichkeit der Aromen im Vergleich zu natürlichem Vanillin aus Vanilleschoten auf einer Skala von 1 (sehr unähnlich) bis 10 (praktisch identisch) beschrieben. Die Ergebnisse sind in Tabelle 1 zusammengefasst; die sensorische Beurteilung stellt den Mittelwert der Testergebnisse dar. Beispiel 1 ist erfinderisch, die Beispiele V1 bis V4 dienen zum Vergleich.

**Tabelle 1**

| Zusammensetzung und Geschmackseindruck; Mengenangaben wenn nicht anders angegeben als ppm | | | | | |
|---|---|---|---|---|---|
| | **V1** | **1** | **V2** | **V3** | **V4** |
| ***Zusammensetzung*** | | | | | |
| Vanillin (Gew.-%) | 93,6 | 92,2 | 93,0 | 93,7 | 93,4 |
| Ferulasäure | 600 | 1.250 | 1.500 | 1.140 | 1.800 |
| 4-HBA | 345 | 37 | 540 | < 10 | 10 |
| Vanillylalkohol | 856 | 290 | 2.940 | 1.060 | 1.100 |
| Vanillinsäure | 260 | 1.880 | 4.580 | 500 | 720 |
| Guajakol | < 10 | 30 | 46 | 60 | 50 |

| ***Sensorische Beurteilung*** | | | | | |
|---|---|---|---|---|---|
| Geschmackseindruck | süß, balsamisch, phenolig | süß, balsamisch, phenolig | süß, phenolig | süß, phenolig | süß, phenolig |
| Ähnlichkeit | 10 | 8,4 | 6,3 | 6,0 | 6,5 |

Vergleichsbeispiel V1 stellt natürliches Vanillin aus Vanilleschoten dar; der Geschmackseindruck dieser Probe diente daher als Maßstab =10. Die Probe 1 wurde nach dem Herstellverfahren H1 gewonnen und ist erfinderisch. Die Probe V2 basiert auf Ferulasäure ex Reiskleie (V2) und wurde nach dem gleichen Verfahren der Erfindung hergestellt. Die beiden Referenzen V3 und V4 wurden biotechnologisch aus Ferulasäure ex (Iso-)Eugenol gewonnen. Man erkennt, dass das Vanillin entsprechend der vorliegenden Erfindung dem Geschmackseindruck der natürlichen Probe am nächsten kommt und einen großen Abstand zu den Vergleichsprodukten aufweist, was auch deshalb besonders überraschend ist, da es den geringsten Vanillingehalt aufweist.

## Patentansprüche

1. Vanillin, dadurch erhältlich, dass man
(a) einen Rückstand aus der Maisverarbeitung mit Alkalien oder esterspaltenden Enzymen behandelt und dabei das darin enthaltende γ-Oryzanol zu Ferulasäure und Sterolen hydrolysiert,
(b) die Ferulasäure aus der Mischung isoliert,
(c) die isolierte Ferulasäure zusammen mit Mikroorganismen vom Typ *Amycolatopsis sp.* DSM 9991 und/oder sp. 9992 in einem Kulturmedium fermentiert, und
(d) nach Beendigung der Reaktion das gebildete Vanillin isoliert.

2. Verfahren zur Herstellung von Vanillin, bei dem man
(a) einen Rückstand aus der Maisverarbeitung mit Alkalien oder esterspaltenden Enzymen behandelt und dabei das darin enthaltende γ-Oryzanol zu Ferulasäure und Sterolen hydrolysiert,
(b) die Ferulasäure aus der Mischung isoliert,
(c) die isolierte Ferulasäure zusammen mit Mikroorganismen vom Typ *Amycolatopsis sp.* DSM 9991 und/oder sp. 9992 in einem Kulturmedium fermentiert, und
(d) nach Beendigung der Reaktion das gebildete Vanillin isoliert.

3. Verfahren nach Anspruch 2, **dadurch gekennzeichnet, dass** man als Rückstand aus der Maisverarbeitung Maiskleie einsetzt.

4. Verfahren nach den Ansprüchen 2 und/oder 3, **dadurch gekennzeichnet, dass** man das im Rückstand aus der Maisverarbeitung enthaltene γ-Oryzanol einer enzymatischen Hydrolyse unterwirft.

5. Verfahren nach mindestens einem der Ansprüche 2 bis 4, **dadurch gekennzeichnet, dass** man die Ferulasäure dem Kulturmedium in solchen Mengen zusetzt, dass eine Konzentration von 60 g/l nicht überschritten wird.

6. Verfahren nach mindestens einem der Ansprüche 2 bis 5, **dadurch gekennzeichnet, dass** man die Ferulasäure dem Kulturmedium nicht auf einmal, sondern portionsweise zusetzt.

7. Verfahren nach mindestens einem der Ansprüche 2 bis 6, **dadurch gekennzeichnet, dass** man ein Kulturmedium einsetzt, welches als Kohlenstoffquelle Zucker, Zuckeralkohole, organische Säuren oder deren Gemische enthält.

8. Verfahren nach mindestens einem der Ansprüche 2 bis 7, **dadurch gekennzeichnet, dass** man ein Kulturmedium einsetzt, welches als Stickstoffquelle einen Hefextrakt enthält.

9. Stoffmischung, enthaltend
(a) Vanillin und
(b) Vanillylvanillat.

10. Stoffmischung nach Anspruch 9, **dadurch gekennzeichnet, dass** sie die Komponenten (a) und (b) im Gewichtsverhältnis 1:99 bis 99:1 enthält.

11. Nahrungsmittel, enthaltend Vanillin erhältlich nach Anspruch 1 oder erhalten nach Anspruch 2.

12. Nahrungsmittel, enthaltend die Stoffmischung nach Anspruch 9.

13. Verfahren zur Aromatisierung von Nahrungsmitteln, bei denen man diesen eine geschmacklich wahrnehmbare Menge Vanillin erhältlich nach Anspruch 1 oder erhalten nach Anspruch 2 oder die Stoffmischung nach Anspruch 9 zusetzt.

14. Verwendung von Vanillin erhältlich nach Anspruch 1 oder erhalten nach Anspruch 2 als Aromakomponente für Nahrungsmittel.

15. Verwendung der Stoffmischung nach Anspruch 9 als Aromakomponente für Nahrungsmittel.
